(19) Europäisches Patentamt
European Patent Office
Office européen des brevets

(11) **EP 4 512 338 A1**

(12) **EUROPEAN PATENT APPLICATION**

(43) Date of publication:
26.02.2025 Bulletin 2025/09

(21) Application number: 23192305.3

(22) Date of filing: 21.08.2023

(51) International Patent Classification (IPC):
*A61B 6/03* (2006.01)   *A61B 6/04* (2006.01)
*A61B 6/00* (2024.01)

(52) Cooperative Patent Classification (CPC):
A61B 6/5264; A61B 5/0037; A61B 5/0077;
A61B 5/055; A61B 5/1128; A61B 5/721;
A61B 5/743; A61B 6/032; A61B 6/0487;
A61B 6/469; A61B 6/527

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• FRERKING, Lena Christina
  Eindhoven (NL)
• SENEGAS, Julien Thomas
  Eindhoven (NL)

(74) Representative: **Philips Intellectual Property &
Standards
High Tech Campus 52
5656 AG Eindhoven (NL)**

(54) **SYSTEM AND METHOD FOR MONITORING A PATIENT**

(57)    A system is provided for monitoring a patient on a moving patient couch during a medical scan. A region of interest is defined of a patent, such as region of the patient that is being scanned at a corresponding point in time. Camera images of the patient are processed to identify the location of the region of interest within the camera images and a modified image of the patient is generated which represents, e.g., highlights, the region of the interest. In this way, movement of the patient that may affect the scan results can more easily be identified, either by manual visual inspection during the scan or by automated detection.

FIG. 1

EP 4 512 338 A1

**Description**

FIELD OF THE INVENTION

This invention relates to the monitoring of a patient, during a medical scan, such as a CT scan.

BACKGROUND OF THE INVENTION

During a CT examination, the patient is typically left alone in the CT room until the completion of the exam. Even though CT scans are rather short in time, multiple scans can be done in sequence, with or without contrast agent administration, so that a typical examination can take up to 15 minutes. During this time, it is highly desirable to continuously monitor the patient and check that she or he feels safe and comfortable and can follow the instructions. It is especially of high importance to make sure that the patient is not moving during the scan, in order to prevent imaging artifacts.

Surveillance cameras can be used to monitor the patient. They are typically located at the ceiling or at the walls, relatively far from the gantry, and with limited visibility to the patient during the scan itself.

The limited visibility means that typical surveillance cameras do not provide additional information to the operator which would help them to better interpret and evaluate the scanning process and the patient situation.

In particular, due to the lens distortion and parallax effect, it is difficult to visualize the exact horizontal and vertical position of the patient with respect to the CT gantry. Therefore, typical surveillance video does not allow to appreciate which body part is currently exactly inside the gantry. Especially, it is not possible for the operator to appreciate which body part travels through scan plane at any point in time.

If a body part is moving during the scan, it would be highly desirable for the operator to detect and visualize whether or not this happens while the body part is traveling through the scan plane.

SUMMARY OF THE INVENTION

The invention is defined by the claims.

According to examples in accordance with an aspect of the invention, there is provided a system for monitoring a patient during a medical scan, wherein the medical scan involves moving the patient on a patient couch relative to a scanning gantry in a patient movement direction, wherein a region of interest is defined of the patient, the system comprising:

a camera for generating camera images of the patient as they move through the scanning gantry; and
a processor for processing the camera images, wherein the processor is configured to:

identify the location of the region of interest within the camera images; and
generate a modified image of the patient, wherein the modification corresponds to the region of interest of the patient.

This system enables a scan operator to identify a patient of interest of patient, so that patient movement of that particular region of interest is more easily identifiable.

The camera is preferably calibrated with respect to the gantry, so that distortions and a slight mismatching of perspective angle of acquired images can be corrected.

The system is for example for use with a CT scanner.

The region of interest can either be defined relative to the patient, hence, moving together with the patient couch, or it can be defined relative to the scanning gantry, hence, static. The region of interest can be any region as long as the observed scene is moving with the patient couch.

In one example, the region of interest comprises a scan region of the patient that is being scanned at a particular point in time. The system thereby enables a scan operator to identify which region of a patient is currently being scanned, so that it can be determined if any patient movement will influence the scan results. Such a scan region may be a scan plane, or any region defined with respect to the scanner coordinates. The region could also move (as a function of time), for example together with the table. For example, this region could be a scan range defined by the operator, or any other anatomical region (head, thorax).

Multiple regions could be shown at the same time, using different colors.

For example, the overall scan volume may be in green (moving with the couch) and the region of interest (e.g., scan region) may be in orange (fixed with respect to the scanner). In this way, the user can see at any time the location of the region of interest, for example which scan parts are entering the scan region.

The camera images preferably comprise pixels, and the processor is configured to:

> obtain a depth map; and
> using the depth map, derive 3D coordinates for each pixel of the camera images relative to the scanning gantry.

The depth map may be part of, or derived from, the images or it may be obtained from a separate source, like a 3D camera used for patient positioning. The depth map could also come from the scan itself.

The combination of the depth map, and the fixed relative position between the gantry and the camera, enables a 3D coordinate map to be derived of the scene captured by the camera.

The processor is for example configured to obtain the depth map by deriving the depth map from the camera images by monitoring the relative displacement, along the patient movement direction, of different pixels between different camera images.

This enables the depth information to be obtained from the camera images themselves without requiring additional depth measurement. The input to the depth analysis is two images that were acquired with the table at two different positions. The corresponding table displacement (e.g., a number in mm) is obtained from the scanning system and is input to the process.

The processor is for example configured to generate the modified image of the patient by modifying the pixels with 3D coordinates within the region of interest. The 3D coordinate map can be compared with the known region of interest (for example when the scan region is positionally fixed relative to the gantry) so that all image pixels which show part of the scene that are within the region of interest can be identified.

The processor is for example configured to generate the modified image by providing a color overlay. This provides an easily visually identifiable indication of the region of the patient that is currently being scanned. Multiple color overlays could be created, each one corresponding to a different region of interest of the patient surface.

A camera pixel row direction or camera pixel column direction is for example parallel to the patient movement direction. This enables movement in the patient movement direction (e.g., horizontal) to be easily identified between successive camera images, and enables movement in an orthogonal direction (e.g., vertical) to be separated. Thus, patient movement due to the patient couch movement can be separated easily from patient motion.

Images (e.g., corrected for perspective distortion) are in this way formed such that the table displacement is along the pixel rows of the image only, so that the correspondence between the two images is found by computing the horizontal shift between corresponding pixels. This results in a disparity measure. The depth is computed from the disparity using the camera intrinsic parameters (e.g., focal length). The correspondence between images is found by state-of-the art algorithms, such as optical flow or cross-correlation, which identify corresponding regions in the pair of images by minimizing a correspondence metric.

The processor is for example further configured to detect motion of the patient at the region of interest. Automatic patient motion detection is thus possible, and targeted to the patient region that is in the region of interest, and which will therefore influence the scan results.

The processor may be further configured to configured to generate the modified image to visualize the location when motion has occurred. Color overlays can for example additionally show the pixel where motion is detected, using a dedicated color.

The processor may be further configured to quantify an amount and/or direction of motion of the patient at the region of interest and to generate the modified image to visualize the amount and/or direction of motion. This may also be achieved using a color scheme. If the motion exceeds a given threshold, the displayed color could indicate a warning signal (for example: red color where the threshold is exceeded).

The processor is for example configured to generate the modified image to visualize the amount and direction of motion using displayed arrows.

The processor may be further configured to modify the camera images to visualize the patient area that has been scanned up to the current point in time or to visualize the patient area that is being scanned at the current point in time.

The invention also provides a method for monitoring a patient during a medical scan, wherein the medical scan involves moving the patient on a patient couch relative to a scanning gantry in a patient movement direction, wherein a region of interest is defined of the patient, the method comprising:

> receiving camera images of the patient as they move through the scanning gantry;
> identifying the location of the region within the camera images; and
> generating a modified image of the patient which represents the region of interest.

The camera images for example comprise pixels, and the method comprises:

> obtaining a depth map; and

using the depth map to derive a 3D coordinate each pixel of the camera images relative to the scanning gantry.

The method may comprise:

detecting motion of the patient at the region of the patient corresponding to the scan plane and modifying the camera images to visualize the location when motion has occurred; or
generating a modified image of the patient, wherein the modification corresponds to the region of interest of the patient.

The invention also provides a computer program comprising computer program code which is adapted, when said program is run on a computer, to implement the method defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows schematically an example of a medical scanner with an imaging system for capturing optical images of the patient during the medical scan;
Fig. 2 shows schematically a patient on a patient couch, to represent a camera image at different couch positions;
Fig. 3 shows the image processing to create a color overlay;
Fig. 4 shows a pinhole camera model;
Fig. 5 shows a pattern used for calibration purposes; and
Fig. 6 shows a camera and its field of view.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for monitoring a patient on a moving patient couch during a medical scan. A region of interest is defined of a patent, such as region of the patient that is being scanned at a corresponding point in time. Camera images of the patient are processed to identify the location of the region of interest within the camera images and a modified image of the patient is generated which represents, e.g., highlights, the region of the interest. In this way, movement of the patient that may affect the scan results can more easily be identified, either by manual visual inspection during the scan or by automated detection.

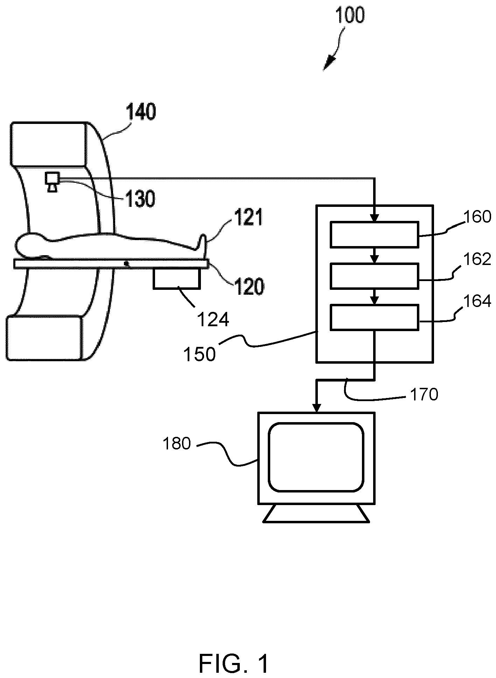

Fig. 1 shows schematically an example of a medical scanner 100 for acquiring a medical image of a subject and which additionally includes an imaging system for capturing optical images of the patient during the medical scan. The images are generated for the purposes of visualizing or automatically detecting patient movement.

The medical scanner 100 comprises a scanning system such as a CT imaging system 140 that is adapted to acquire a medical image, i.e., a CT image in this example, of a patient 121 positioned on a patient support 120. The patient support 120 is adapted to move the patient 121 through the CT imaging system 140 during the CT imaging procedure. For this purpose, the medical scanner has a drive system 124 for driving the patient support through the scanning system.

The imaging system comprises an optical camera 130 that is adapted to acquire monitoring images of the patient 121 during the CT imaging procedure, i.e., while they move through the scanning gantry, and a processor 150. The operation of the system with a single camera will be explained, but there may be multiple cameras. The camera can be a color or monochrome camera. It can use visible light or infrared light. The camera is calibrated with respect to the scanner, i.e. the scanner gantry.

The camera may be mounted on the gantry as shown, but it may be mounted in a fixed position relative to the couch. In all cases, the position of the camera relative to gantry needs to be known. If mounted relative to the couch, he movement of the couch may be used to derive the position of the camera relative to the gantry, and hence relative to the scanning region of the scanner.

The camera 130 has a wide field of view so that it captures a full view of the patient support 120, or at least the part of the patient support 120 over which regions of interest of the patient will be positioned.

The camera for example comprises a fisheye lens with a wide field of view. The patient support (and the patient on the patient support) can be imaged either by a single camera as shown or by a small set of cameras. The wide angle lens results in image distortion, such that the shape of objects appears differently at different regions of the field of view of the camera. As a result, the shape of objects or regions of interest will change when the position of those objects or regions of interest change in the field of view of the camera, as a result of the movement of the patient support.

To address this issue, the processor 150 performs image post-processing to correct those distortions within the captured images, i.e., resulting from the width of the field of view of the camera. This is shown as post-processing unit 160. For the purposes of the post-processing, the cameras are calibrated, such that geometric distortions are corrected by the post-processing, and the position and orientation of each camera with respect to the scanner coordinate system is known.

In accordance with the invention, the processor identifies the location of the a region of interest within the camera images, in step 162. A modified image of the patient is generated in step 164, wherein the region of interest is modified.

In one example, the region of interest is the region of the patient that lies within the scan region. Thus, it is a different region of the patient as the patient couch moves through the gantry. In this case, the image modification is used to show which part of the patient is in the scan region. In another example, the region of interest is a fixed region of the patient (e.g., the face). In this latter case, the location of the region of interest (e.g. the face or a location where a needle is placed) is highlighted during the couch motion.

The modified image sequence is provided as output 170 for display on a display 180. This display 180 may be part of the medical scanner, or it may be a separate device such as a mobile device of a technician or it may be part of a remote operation center, to which the image data is transmitted by wired or wireless data transfer.

The output 170 comprises a continuous video stream. There may be a video stream of one or more regions of interest.

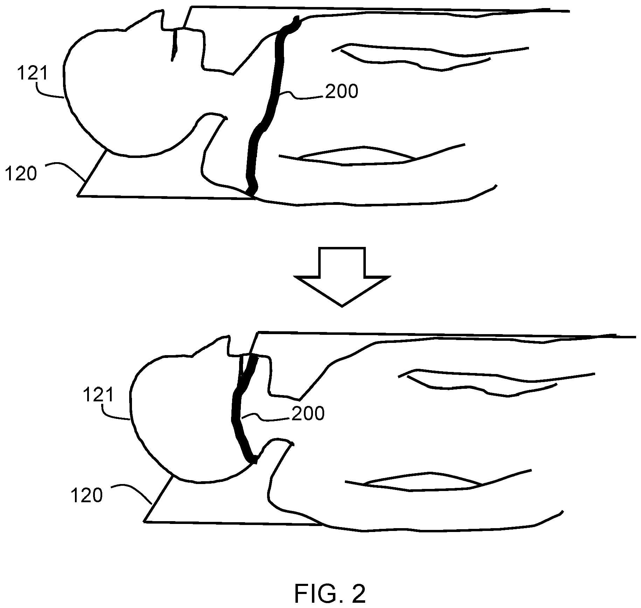

Fig. 2 shows schematically the patient 121 on the patient couch 120, to represent a camera image. In this example, the image modification comprises a color overlay 200 on top of the video stream to identify and to visualize the exact region of the patient that is currently in the imaging area, i.e., the scan plane. The modification is shown for two different time frames, with patient couch movement between them.

Identifying the projections of the scan plane region on the image data can be used to detect and quantify motion events occurring during the scan only at those locations where it has impact on the scan data.

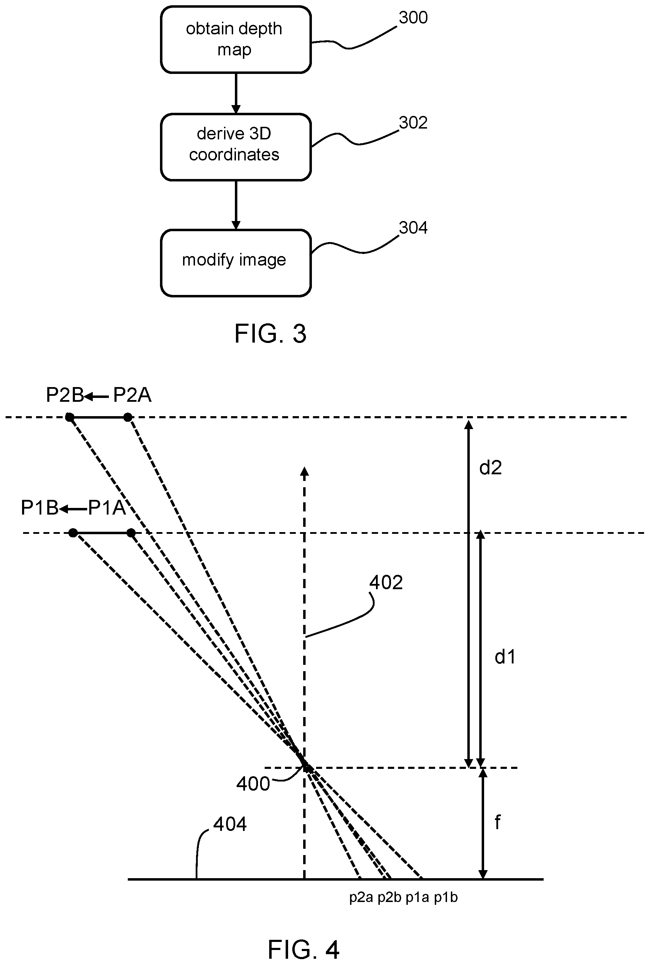

Each incoming frame from the camera is processed in real-time using the method of Fig. 3:

As shown in Fig. 3, the image processing involves obtaining a depth map in step 300 and in step 302 using the depth map to derive 3D coordinates for each pixel of the camera images relative to the scanning gantry. This involves use of the camera calibration parameters. All pixels whose 3D coordinates are comprised within the region of interest (e.g., CT scan plane for this particular example) are identified, and for those pixels, the color overlay is provided in step 304.

The depth map may be obtained from a separate source (e.g., a depth camera), such as a the 3D camera or depth camera used to position the patient. Another option is to use the acquired CT scan, especially the 3D surview scan acquired at the very beginning of the exam. The outer body surface can be segmented from the CT scan and converted to 3D point cloud Another option is to derive the depth map from the sequence of successive images from the same camera used to capture the patient images. This is discussed further below.

In one preferred example, the camera is mounted at the gantry of the scanner in a way that the x-axis (the pixel row direction of the camera) is approximately parallel to the couch travel direction. By using the intrinsic and extrinsic calibration parameters of the camera, distortions and a slightly mismatching perspective angle of acquired images can be corrected such that the table movement takes place perpendicular to the optical axis.

Analyzing different frames of the acquired video between which the table has been moving creates a stereo effect. Applying a motion detection algorithm, e.g., optical flow, allows motion to be separated that takes place in horizontal direction from motion in vertical direction.

As mentioned above, the depth map may be derived from the images themselves. This is explained with reference to a pinhole camera model as shown in Fig. 4. The optical center is shown as 400, the optical axis is 402 and the image plane is 404 at a focal distance f from the optical center. The relative displacement for points located at different depths d1 and d2 to the optical center is scaled and the scaling factor only depends on the depth ratio.

Points P1A and P1B at depth d1 are imaged to points p1a and p1b. Points P2A and P2B at depth d2 are imaged to points p2a and p2b.

The scaling leads to the effect that the apparent motion in regions that are further away from the camera is smaller than the apparent motion close to the camera, even though the true motion is the same. Hence, the measured disparity in horizontal direction together with the known actual table movement in between the frames enables a depth map to be generated of the given view.

The layout of Fig. 4 yields:

$$\frac{d_1}{f} = \frac{\overline{P_{1A}P_{1B}}}{\overline{p_{1a}p_{1b}}} \; and \; \frac{d_2}{f} = \frac{\overline{P_{2A}P_{2B}}}{\overline{p_{2a}p_{2b}}}$$
$$\rightarrow \overline{p_{2a}p_{2b}} = \frac{d_1}{d_2} \cdot \overline{p_{1a}p_{1b}}$$

From the depth map and the focal length of the camera, the 3D coordinates of the visible pixels can be determined for incoming video frames with associated table motion. The pixels of the moving parts that are within a given region of interest, like the scan plane, can thereby be identified.

These pixels can be further analyzed with respect to motion events by tracking the calculated motion in the vertical (pixel column) direction. Since motion in this direction is not related to table motion, it can certainly be determined as motion from the patient, which may lead to artefacts in the scan data.

The detected motion in a given area can also be quantified. From the known table movement and the measured flow in the horizontal direction, the resolution of the individual pixels can be identified, i.e., the quantitative distance of the measured flow per pixel is known. This resolution per pixel is the same for horizontal and vertical motion. Therefore, also the absolute distance in vertical direction can be obtained, which means that it can be determined how many mm. for example a head is lifted during an exam.

Thus, to summarize, for each pixel, the horizontal and vertical shifts between two images acquired at two consecutive time points may be computed using means known from state of the art, like optical flow or cross-correlation. The horizontal shift is due to the table displacement. The vertical shift is due to patient motion, if present. The amplitude in pixels of the horizontal shift depends on the depth of the corresponding part with respect to the camera: the closer the part is to the camera, the larger the pixel shift will be. In other words, together with the value of the table displacement between the two images, the amplitude of the horizontal shifts provides the physical optical resolution of the camera at this location. It can hence be used to compute the physical amplitude of the vertical shift. This last step is simply obtained by dividing the vertical pixel shift by the horizontal pixel shift, and multiplying this quotient by the table displacement in millimeters.

In other examples, other interesting regions related to the CT gantry geometry or to the scan field of view could be shown as overlay. For example, the whole scan range defined during planning could be shown in real-time on each frame, so that the operator can visualize the progress of the scan.

For a proper visualization of the overlays, it is desirable to generate overlays which are consistent over time, i.e., do not flicker from frame to frame due to noise in the depth maps. A way to improve the temporal smoothness is to generate an average depth map from multiple, single-frame based depth maps.

In this way, temporal filtering of the depth map may be achieved by computing a new depth map for each video frame, or averaging the depth map, or using the same depth map for several consecutive frames (i.e., a sliding window mode). The latter approach has the advantage that it produces smooth overlays from frame to frame.

By way of example, the following steps may be applied for this purpose. For each input frame, the 3D coordinates are computed using the computed depth map. All 3D point clouds are translated to a reference position using the table motion corresponding to each input frame The 3D points are then merged and averaged or filtered in other way. Outliers are then removed. The 3D point cloud is reprojected as the depth map for each input frame taking into account the input table motion for this frame.

The example above provides a visual overlay of the area of the patient that is within the scan region. As mentioned above, another option is to provide a visual representation of a particular region of the patient. Thus, one option is to provide an overlay of the scan plane (corresponding to the collimator width) which remains fixed with respect to the gantry. Another option is to provide an overlay of a scan region of the patient (i.e., a region of the patient within the overall scan being performed) which moves with the couch. Another option is to provide an overlay of any body part of the patient, even if not scanned, like the face, which moves with the couch.

Other additional information may also be visualized.

As mentioned above, patient movement may be detected automatically. The image modification may then also visualize the location when motion has occurred, for example with a flashing part of the color overlay, or a different color, or other way of highlighting that particular portion of the image. The amount and/or direction of motion of the patient at the region of the patient corresponding to the scan region may also be quantified as explained above. The image modification may also visualize the amount and/or direction of motion, for example using displayed arrows (with a length representing an amount of movement and a direction representing the direction of movement). If the motion exceeds a given threshold, the displayed color could indicate a warning signal (for example a red color where threshold is exceeded).

The image modification may also visualize the patient area that has been scanned up to the current point in time instead of only the patient area that is being scanned at the current point in time as explained above.

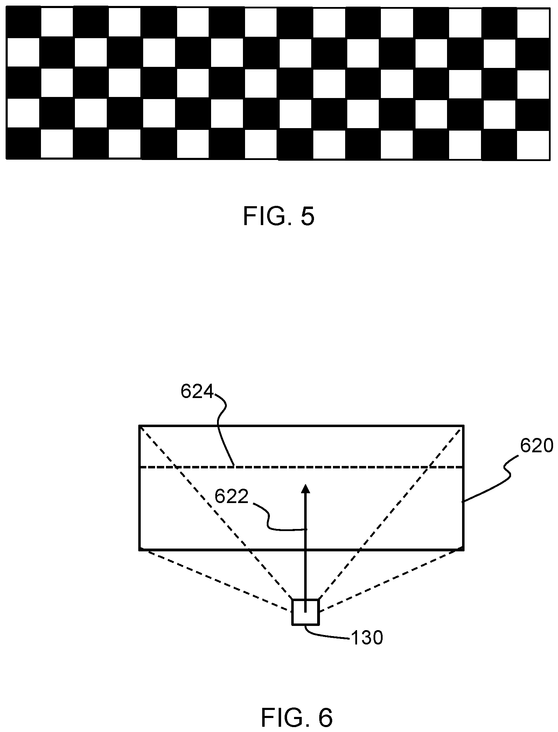

As explained above, a calibration process is used to derive the post-processing requirements to correct for image distortion. One approach is to apply a known pattern to the patient support, such as the checkerboard pattern shown in Fig. 5. Image distortions, particularly prominent at the edges of the field of view, may be corrected be deriving a correction function which returns the captured images to the known "correct" image.

As briefly explained above, the camera may be oriented in such a way to make movement identification simpler.

Fig. 6 shows a camera 130 and its field of view 620, configured in the manner discussed above. The camera has a central axis 622 which extends to a center of the field of view. This is the optical axis of the camera. This optical axis 622 is for example a vector which is normal to the plane of an array of image sensing elements and projects from a center of the array of imaging sensing elements.

The optical axis 622 is orthogonal to a direction of the patient support displacement in use of the scanner, e.g., the length direction of a patient support in the form of a couch. Furthermore, pixel rows of images captured by the camera correspond to locations along an axis parallel to the direction of patient support movement. A line 624 across the field of view maps to a pixel row in the generated image, and this line 624 within the field of view (e.g. when the camera is imaging the empty patient support) is parallel to the direction of patient support displacement, i.e. parallel to the length direction of the patient support.

This defines a particular orientation of the camera relative to the patient support. It means that in a captured image of an empty patient support, the pixel rows correspond to horizontal lines along the length of the patient support, whereas the pixel columns include a vertical component.

Thus, movement may be automatically measured by analyzing changes in location of the patient in the pixel column direction, as between sequential captured images.

For this purpose, the camera should not be directly overhead, since it will not then capture vertical displacements. Two cameras may for example capture partially lateral views of the patient, so that vertical patient movement will result in a component of movement in the pixel column direction of the captured images. There may be one or more cameras at one side of the gantry only, or there may be one or more cameras at each side of the gantry.

This imaging system thereby captures images in which patient support movement results in displacement in the pixel row direction whereas typical patient movement, such as breathing, is in an orthogonal direction, predominantly in the vertical direction.

As mentioned above, a calibration procedure is used. The output of the calibration process is a set of camera intrinsic parameters describing the optical properties of the sensor and length, such as focal length, optical center, and distortion coefficients. These parameters can then be used as input to compute the distortion free images. The calibration process also allows computing the exact position and orientation of the camera with respect to the scanner coordinate system, which is typically linked to the isocenter of the scanner. This step is referred to as computing the extrinsic parameters of the camera (pose matrix) and allows correcting for the perspective effect.

Based on the extrinsic parameters, the spatial coordinates of the moving parts can be expressed in scanner coordinate systems. Especially, this allows to detect whether the motion is taking place at or in the vicinity of the scan plane.

Based on the location and amplitude of the patient movement in the one or more regions of interest, an overall motion signal can be derived for the time of the scan, and significant patient motion can thus be detected. This computed motion signal can then be used to inform the operator about patient motion, either prior to or during the scan. It can be used to predict which slices of the imaged volume will be impacted by motion. It can also be used as input for the reconstruction of the data (e.g., gating or motion correction) and for 4D CT scans.

The invention may be applied to any medical scanner in which a patient support is moved during the imaging procedure, like a PET imaging device, an MR imaging device, a SPECT imaging device, as well as a CT scanner as described above. The medical scanner may comprise a C-arm or a closed bore. The camera or cameras may be located in the bore or at an inner surface of the C-arm, or outside the bore or C-arm envelope. In preferred examples, the camera is static relative to the main body of the scanning system and thus the patient support moves relative to the camera. The camera may not be mounted directly to the medical scanner but may instead be in a fixed position relative to the medical scanner by a separate mounting. However, the camera may instead move with the patient couch since its motion can be compensated.

Although in the above described embodiments the patient support is always a patient support on which a patient is lying during the acquisition of the medical image, the patient support can also be configured for a sitting or a standing patient.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1.  A system for monitoring a patient during a medical scan, wherein the medical scan involves moving the patient on a patient couch relative to a scanning gantry in a patient movement direction, wherein a region of interest is defined of the patient, the system comprising:

    a camera (130) for generating camera images of the patient as they move through the scanning gantry; and
    a processor (150) for processing the camera images, wherein the processor is configured to:

    (302) identify the location of the region of interest within the camera images; and
    (304) generate a modified image of the patient, wherein the modification corresponds to the region of interest of the patient.

2.  The system of claim 1, wherein the region of interest comprises a scan region of the patient that is being scanned.

3.  The system of claim 1 or 2, wherein the camera images comprise pixels, and wherein the processor is configured to:

    obtain a depth map;
    optionally perform temporal filtering of the depth maps over time; and
    using the depth map, derive 3D coordinates for each pixel of the camera images relative to the scanning gantry.

4.  The system of claim 3, wherein the processor is configured to obtain the depth map by deriving the depth map from the camera images by monitoring the relative displacement, along the patient movement direction, of different pixels between different camera images.

5.  The system of claim 3 or 4, wherein the processor is configured to generate the modified image of the patient by modifying the pixels with 3D coordinates within the region of interest, for example by providing a color overlay.

6.  The system of any one of claims 1 to 5, wherein a camera pixel row direction or camera pixel column direction is parallel to the patient movement direction.

7.  The system of any one of claims 1 to 6, wherein the processor is further configured to detect motion of the patient at the region of interest.

8.  The system of claim 7, wherein the processor is further configured to generate the modified image to visualize the location when motion has occurred.

9.  The system of claim 8, wherein the processor is further configured to quantify an amount and/or direction of motion of the patient at the region of interest and to generate the modified image to visualize the amount and/or direction of motion.

10. The system of claim 9, wherein the processor is configured to generate the modified image to visualize the amount and direction of motion using displayed arrows.

11. The system of any one of claims 1 to 10, wherein the processor is further configured to generate the modified image to:

    visualize the patient area that has been scanned up to the current point in time;
    or
    visualize the patient area that is being scanned at the current point in time.

12. A method for monitoring a patient during a medical scan, wherein the medical scan involves moving the patient on a patient couch relative to a scanning gantry in a patient movement direction, wherein a region of interest is defined of the patient, the method comprising:

> receiving camera images of the patient as they move through the scanning gantry;
> identifying the location of the region of interest within the camera images; and
> generating a modified image of the patient, wherein the modification corresponds to the region of interest of the patient.

13. The method of claim 11, wherein the camera images comprise pixels, and wherein the method comprises:

> obtaining a depth map;
> optionally performing temporal filtering of the depth maps over time; and
> and
> using the depth map to derive a 3D coordinate each pixel of the camera images relative to the scanning gantry.

14. The method of claim 12, comprising:

> detecting motion of the patient at the region of interest and modifying the camera images to visualize the location when motion has occurred; or
> modifying the camera images to visualize the location of the region of interest during the couch motion.

15. A computer program comprising computer program code which is adapted, when said program is run on a computer, to implement the method of any one of claims 11 to 14.

FIG. 1

FIG. 2

obtain depth
map                    300

derive 3D
coordinates            302

modify image           304

FIG. 3

P2B ← P2A

P1B ← P1A

d2

402

d1

400

404

f

p2a p2b p1a p1b

FIG. 4

FIG. 5

FIG. 6

Europäisches
Patentamt

European
Patent Office

Office européen
des brevets

# EUROPEAN SEARCH REPORT

**Application Number**

**EP 23 19 2305**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/358676 A1 (SENEGAS JULIEN [DE]) 10 November 2022 (2022-11-10) * paragraphs [0040], [0041], [0042]; figure 3 * | 1-15 | INV. A61B6/03 A61B6/04 A61B6/00 |
| X | US 2020/205748 A1 (PAUTSCH ADAM GREGORY [US] ET AL) 2 July 2020 (2020-07-02) * paragraph [0052] – paragraph [0072]; figures 5A,5B, 6 * | 1-15 | |

**TECHNICAL FIELDS SEARCHED (IPC)**

A61B
A61N

The present search report has been drawn up for all claims

| Place of search | Date of completion of the search | Examiner |
|---|---|---|
| Munich | 20 December 2023 | Marzal-Abarca, X |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 19 2305**

This annex lists the patent family members relating to the patent documents cited in the above-mentioned European search report.
The members are as contained in the European Patent Office EDP file on
The European Patent Office is in no way liable for these particulars which are merely given for the purpose of information.

**20-12-2023**

| Patent document cited in search report | | | Publication date | Patent family member(s) | | | Publication date |
|---|---|---|---|---|---|---|---|
| US 2022358676 | A1 | | 10-11-2022 | CN | 114269236 | A | 01-04-2022 |
| | | | | EP | 3783379 | A1 | 24-02-2021 |
| | | | | EP | 4018215 | A1 | 29-06-2022 |
| | | | | JP | 7391185 | B2 | 04-12-2023 |
| | | | | JP | 2022545663 | A | 28-10-2022 |
| | | | | US | 2022358676 | A1 | 10-11-2022 |
| | | | | WO | 2021032606 | A1 | 25-02-2021 |
| US 2020205748 | A1 | | 02-07-2020 | CN | 111374675 | A | 07-07-2020 |
| | | | | EP | 3673806 | A2 | 01-07-2020 |
| | | | | JP | 7242514 | B2 | 20-03-2023 |
| | | | | JP | 2020121104 | A | 13-08-2020 |
| | | | | US | 2020205748 | A1 | 02-07-2020 |

EPO FORM P0459

For more details about this annex : see Official Journal of the European Patent Office, No. 12/82